# EUROPEAN PATENT SPECIFICATION

(11) **EP 2 814 576 B1**
(45) Date of publication and mention of the grant of the patent: **15.11.2017**
(21) Application number: 13715549.5
(22) Date of filing: 26.03.2013
(51) Int. Cl.: A61Q 5/12, C07C 215/40

(54) **POLYCATIONIC POLYHYDROXYL COMPOUNDS**
POLYKATIONISCHE POLYHYDROXYLVERBINDUNGEN
COMPOSÉS POLYHYDROXYLE POLYCATIONIQUES

(30) Priority: 30.03.2012 US 201261618009 P
(43) Date of publication of application: 24.12.2014
(73) Proprietor: Dow Global Technologies LLC, Midland, MI 48674 (US)
(72) Inventor: DEAVENPORT, Joseph, L., Lake Jackson, TX 77566 (US)
(74) Representative: Houghton, Mark Phillip
(86) International application number: PCT/US2013/033777
(87) International publication number: WO 2013/148613

(56) References cited:
- EP-A1- 0 634 468
- DE-B1- 2 538 745
- JP-A- 2011 236 187
- US-A1- 2007 048 235
- US-A1- 2007 102 363
- BURIKS ET AL: "Intramolecular Cyclization Products from Alkanolamines and Epichlorohydrin", JOURNAL OF ORGANIC CHEMISTRY, ACS, US, vol. 52, no. 23, 1 November 1987 (1987-11-01), pages 5247-5254, XP008153559, ISSN: 0022-3263, DOI: 10.021/JO00232A035
- DATABASE CA [Online] CHEMICAL ABSTRACTS SERVICE, COLUMBUS, OHIO, US; KIMSANOV, B. KH ET AL: "Prospects for development of syntheses based on glycerol", XP002698934, retrieved from STN Database accession no. 2000:433558 -& KIMSANOV, B. KH ET AL: "Prospects for development of syntheses based on glycerol", BASHKIRSKII KHIMICHESKII ZHURNAL , 7(1), 8-15 CODEN: BKZHFU; ISSN: 0869-8406, 2000, XP008163043,
- CHU, QI ET AL: "Synthesis, adsorption and wettability of organic polyhydroxy triammonium salt as molecular deposition filming/flooding agent", YOUTIAN HUAXUE , 25(3), 281-283, 296 CODEN: YHOUA3; ISSN: 1000-4092, 2008, XP008163042,

## Description

### Field

The present invention relates to novel polycationic polyhydroxyl compounds and their uses in personal care compositions.

### Background

Polyhydroxyl compounds, or polyols, have a number of uses, from raw materials used in the manufacture of urethane foams to humectants for personal care products like shaving foams, skin and sun care lotions and sprays, hair conditioning formulations, hair color formulations, make-up formulations, anti-wrinkle formulations, formulations that promote hair volume or shine, hair styling products such as those in the mousse, lotion, wax, aerosol, or gel form for fixing a hair style, skin and facial cleansing liquids, bars, and foams, hair removers, sunscreens, and shampoos.

Quaternary ammonium compounds are also useful in a number of applications, such as for disinfectants, surfactants, fabric softeners, and conditioners in shampoos. US 2007/0048235 discloses compositions comprising a quaternised nitrogen moisturizing agent.

Despite the number of available conventional compounds, there is a strong need for novel compounds with properties to differentiate performance or offer synergistic effects in areas of interest, particularly in personal care compositions.

### Detailed Description

In one embodiment, the present invention provides compounds, including salts, of the Formula (I): 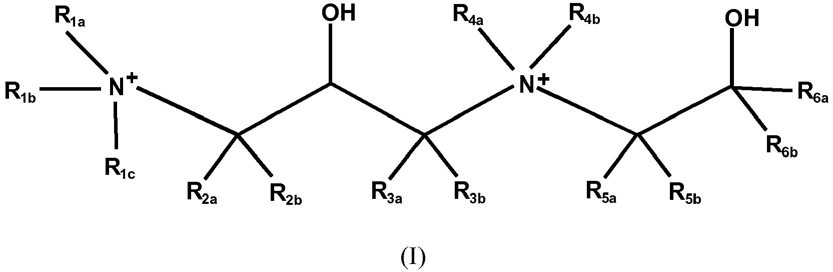 wherein:
R₁ₐ, R_{1b}, R_{1c}, R₂ₐ, R_{2b}, R₃ₐ, R_{3b}, R₅ₐ, R_{5b}, R₆ₐ, and R_{6b}, are, independently, H,
optionally substituted C1-C6 alkyl, or R₂ₐ and R_{2b}, R₃ₐ and R_{3b}, or R₅ₐ and R_{5b}, may cooperate to form a cycloalkyl; and
R₄ₐ and R_{4b} are each -CH₂CH(OH)CH₂OH, or R₄ₐ is -(CH₂)₃CH₃ and R_{4b} is -CH₂CH₂OH,
provided that at least one of R₄ₐ, R_{4b}, R₅ₐ, R_{5b}, R₆ₐ, and R_{6b} is C1-C6 alkyl substituted with at least one hydroxy group or amino group
wherein the term "optionally substituted" means that the groups in question are either unsubstituted or substituted with one or more groups, radicals or moieties, selected from halogen, hydroxy, amino or carboxy, and "amino" includes amino further substituted with C1-C3alkyl.

"Amino" is preferably trimethylamino (-N⁺(CH₃)₃).
When the groups in question are substituted with more than one substituent, the substituents may be the same or different. In one embodiment, the optional substituent is selected to produce a cosmetically acceptable compound. "Cosmetically acceptable" refers to ingredients typically used in personal care compositions, and is intended to underscore that materials that are toxic, irritating, or unpleasant smelling when present in the amounts typically found in personal care compositions are not contemplated as part of the present invention. In one embodiment, the optional substituent is one or more hydroxy groups.

"Alkyl" means a saturated monovalent linear or branched aliphatic hydrocarbon radical. Representative examples include, but are not limited to methyl, ethyl, propyl, isopropyl, butyl, isobutyl, tert-butyl, sec-butyl, pentyl, isopentyl, neopentyl, hexyl, isohexyl, and the like.

The term "cycloalkyl" denotes a saturated monocyclic or bicyclic cycloalkyl group. Examples of cycloalkyl groups include cyclopropyl, cyclobutyl, cyclopentyl, cyclohexyl, cycloheptyl, cyclooctyl, and the like. In one embodiment, the cycloalkyl is cyclohexyl or cyclopentyl.

Salts means that a counter-ion is present, preferably halogen, more preferably Cl⁻.

In one embodiment, R₁ₐ, R_{1b}, R_{1c}, are each methyl.

In one embodiment, R₂ₐ, R_{2b} R₃ₐ, and R_{3b} are each H.

The present invention further provides compounds, including salts, of the Formula (II): 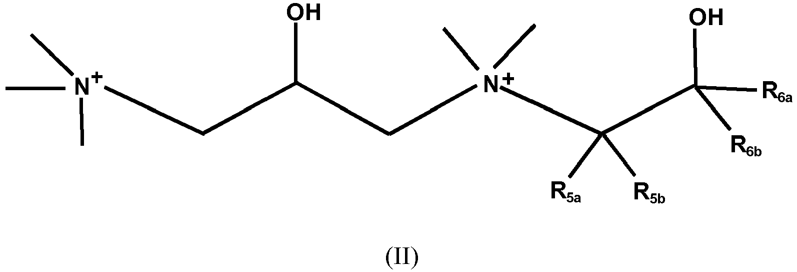 wherein:
R₅ₐ is ethyl, R_{5b} is -CH₂OH, and R₆ₐ and R_{6b} are each H.

In one embodiment, the present invention provides methods for providing humectancy in a personal care composition, comprising including the compound of Formula I into the personal care composition. The ingredients used, and their proportions and manner of addition, are familiar to those versed in conventional personal care compositions, including, optionally, cosmetically acceptable emollients and moisturizers such as for example glycerin, mineral oil/petrolatum, propylene glycol, sorbitol, polyethylene glycol, conditioners such as for example quaternary ammonium conditioners, polyquaternium conditioners such as polyquaternium-10, 67, polyolefins, guar hydroxypropyltrimonium chloride, oils such as triglyceride oils, silicone oils, hydrocarbon oils, waxes, sunscreen UV filters, surfactants, emulsifiers, preservatives, rheology modifiers, texture modifiers such as silicones or silicone elastomers, polyglycols, HEC, HPMC, colorants or pigments for the hair or skin, preservatives, pH adjustors, propellants, reducing agents, fragrances, foaming or defoaming agents, tanning agents, depilatory agents, astringents, antiseptics, deodorants, antiperspirants, insect repellants, bleaches, lighteners, anti-dandruff agents, adhesives, polishes, strengtheners, fillers, barrier materials, hair fixative film forming polymers such as, for example those under the INCI name of acrylates/hydroxyesters acrylates copolymer or styrene/acrylates copolymer or biocides.

In one embodiment, the present invention provides hair care compositions containing the compound of Formula I or Formula II.

In one embodiment, the present invention provides skin care compositions containing the compound of Formula I or Formula II.

In one embodiment, the present invention provides sun care compositions containing the compound of Formula I or Formula II.

### Examples

The following examples are for illustrative purposes only and are not intended to limit the scope of the present invention.

### Reference Example 1

### Reaction of bis(3-chloro-2-hydroxypropyl)dimethylammonium chloride and trimethylamine to afford 2-hydroxy-N¹-(2-hydroxy-3-(trimethylammonio)propyl)-N¹, N¹, N³, N³, N³-pentamethylpropane-1,3-diaminium chloride

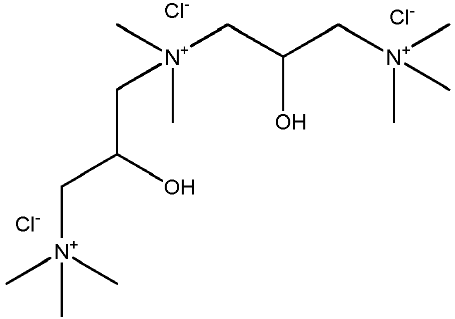

1.6253 g of bis(3-chloro-2-hydroxypropyl)dimethylammonium chloride (42.5% solution in water) was added to a glass vial. 1.1760 g trimethylamine (43.6% solution in water) was added to the vial and the solution was stirred at ambient temperature for 16 hours. The solution was heated to 60 °C for 4 hours.
¹³C NMR spectra acquired from a Bruker 300MHz spectrometer (samples prepared as ∼30 wt% in D₂O) confirmed the title compound: DEPT NMR (250 MHz, D20) (52.9, 54.6, 62.0, 66.5, 67.7)

### Example 2

### Reaction of 2,3-epoxypropyltrimethylammonium chloride and dimethylaminoethylpropanediol to afford 2-hydroxy-N¹-(1-hydroxy-2-(hydroxymethyl)butan-2-yl)-N¹, N¹, N³, N³, N³-pentamethylpropane-1,3-diaminium chloride

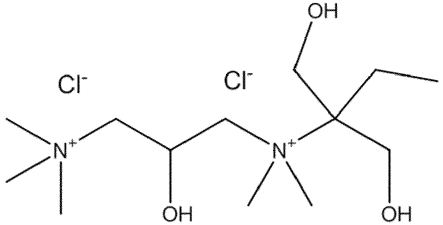

A 250 mL round bottom, jacketed, flask was equipped with a magnetic stirbar and placed on a stir plate. The temperature was set to 13 °C and monitored with a thermocouple. 3-chloro-2-hydroxypropyltrimethylammonium chloride (Quat 188, 69%, commercially available from The Dow Chemical Company), 51.63g (0.19 mol), was added to the reactor flask. Next, 15.87g sodium hydroxide (49.8%, 0.20 mol), was added dropwise over about 10 minutes while maintaining a temperature of about 15 °C.

The solution was allowed to stir for one hour at 13 °C. The temperature was then set to 4 °C and 23.26g of dimethylaminoethylpropanediol (0.16 mol) was added. The temperature was set to 45 °C and the reaction mixture was allowed to stir for three hours whereupon the pH was decreased to 7 by adding 33.47 g hydrochloric acid (13%). NMR confirmed reaction completion.
¹³C NMR spectra acquired from a Bruker 300MHz spectrometer (samples prepared as ∼30 wt% in D20) confirmed the title compound: DEPT NMR (250 MHz, D20) 9.9, 23.1, 41.1, (56.7, 57.0), 62.3, 64.7, 66.1, 70.6.

### Reference Example 3

Compounds prepared substantially according to Reference Example 1 and Example 2 are made and formulated into personal care compositions having otherwise conventional ingredients. The compositions are evaluated by trained panelists, with each panelist being asked to compare the inventive compositions to a conventional composition.

For hair care compositions, wet and dry feel preference and wet and dry combability is measured by asking the panelists to feel and comb two hair tresses of European virgin brown hair, commercially available from International Hair Importers and Products Inc. NY (USA), one hair tress treated with an inventive composition, the other hair tress treated with a conventional composition. Each panelist is asked to compare the tresses and state which tress is smoother to comb / feel. The answer "same" is not allowed. The reported number is the percent of panelists preferring one over the other.

For skin care compositions, panelists apply a sample (one inventive composition, one conventional composition) to a designated area on their right or left forearm. Initially, each sample is evaluated for ease of application, play time, evenness of deposit, coverage, speed of adsorbtion, shine, matte, skin moistness, heaviness, amount of grease, amount of tack, quickness of drying, overall skin feel, and overall appearance. After a designated time, each sample is again evaluated, this time for coverage, evenness of coverage, shine, matte, skin moistness, heaviness, and overall appearance.

## Claims

1. A compound, including salts, of the Formula (I): 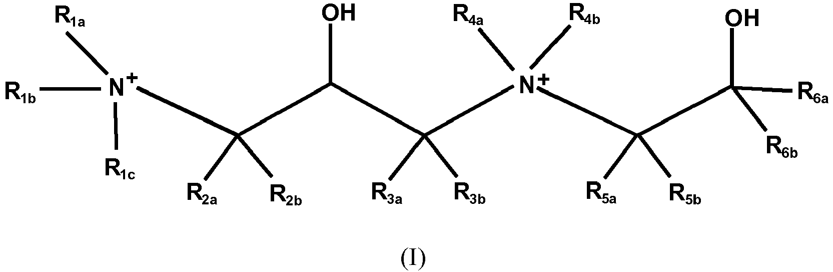 wherein:
R₁ₐ, R_{1b}, R_{1c}, R₂ₐ, R_{2b}, R₃ₐ, R_{3b}, R₅ₐ, R_{5b}, R₆ₐ, and R_{6b}, are, independently, H,
optionally substituted C1-C6 alkyl, or R₂ₐ and R_{2b}, R₃ₐ and R_{3b}, or R₅ₐ and R_{5b}, may cooperate to form a cycloalkyl; and
R₄ₐ and R_{4b} are each -CH₂CH(OH)CH₂OH, or R₄ₐ is -(CH₂)₃CH₃ and R_{4b} is - CH₂CH₂OH,
provided that at least one of R₄ₐ, R_{4b}, R₅ₐ, R_{5b}, R₆ₐ, and R_{6b} is C1-C6 alkyl substituted with at least one hydroxy group or amino group
wherein the term "optionally substituted" means that the groups in question are either unsubstituted or substituted with one or more groups, radicals or moieties, selected from halogen, hydroxy, amino or carboxy, and "amino" includes amino further substituted with C1-C3alkyl.

2. The compound of Claim 1, wherein R₁ₐ, R_{1b}, and R_{1c} are each methyl.

3. The compound, including salts, having the Formula (II): 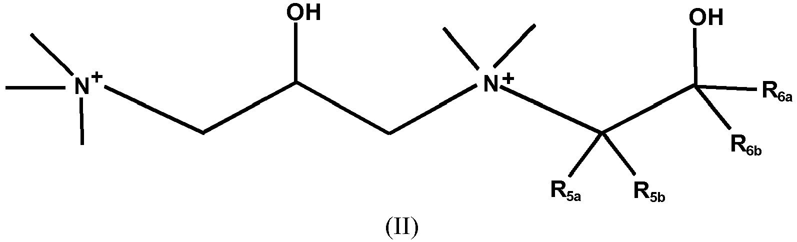 wherein:
R₅ₐ is ethyl, R_{5b} is -CH₂OH, and R₆ₐ and R_{6b} are each H.

4. A method for providing humectancy in a personal care composition, comprising adding of the compounds of claims 1-3 into the personal care composition.

5. A hair care composition containing any of the compounds of claims 1-3.

6. A skin care composition containing any of the compounds of claims 1-3.

7. A sun care composition containing any of the compounds of claims 1-3.

## Patentansprüche

1. Eine Verbindung, einschließlich Salzen, der Formel (I): 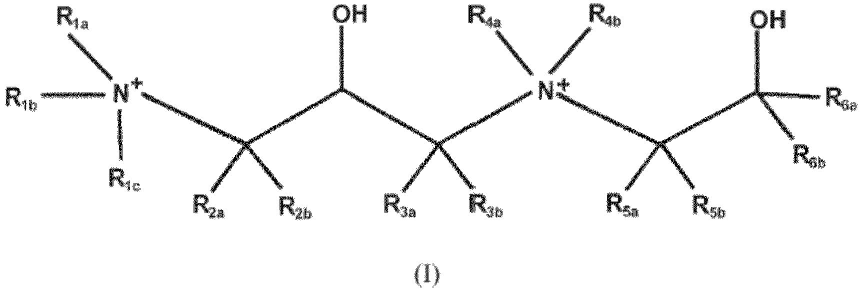 wobei:
R₁ₐ, R_{1b}, R_{1c}, R₂ₐ, R_{2b}, R₃ₐ, R_{3b}, R₅ₐ, R_{5b}, R₆ₐ und R_{6b} unabhängig H, optional substituiertes C1-C6-Alkyl sind oder R₂ₐ und R_{2b}, R₃ₐ und R_{3b}, oder R₅ₐ und R_{5b} zusammenwirken können, um ein Cycloalkyl zu bilden; und
R₄ₐ und R_{4b} jeweils -CH₂CH(OH)CH₂OH sind oder R₄ₐ -(CH₂)₃CH₃ ist und R_{4b} -CH₂CH₂OH ist,
mit der Maßgabe, dass mindestens eines von R₄ₐ, R_{4b}, R₅ₐ, R_{5b}, R₆ₐ und R_{6b} C1-C6-Alkyl, substituiert mit mindestens einer Hydroxygruppe oder Aminogruppe, ist,
wobei der Begriff "wahlweise substituiert" bedeutet, dass die besagten Gruppen entweder nichtsubstituiert oder mit einer oder mehreren Gruppen, Resten oder Teilen, ausgewählt aus Halogen, Hydroxy, Amino oder Carboxy, substituiert sind, und "Amino" ferner mit C1-C3-Alkyl substituiertes Amino umfasst.

2. Verbindung gemäß Anspruch 1, wobei R₁ₐ, R_{1b} und R_{1c} jeweils Methyl sind.

3. Verbindung, einschließlich Salzen, mit der Formel (II): 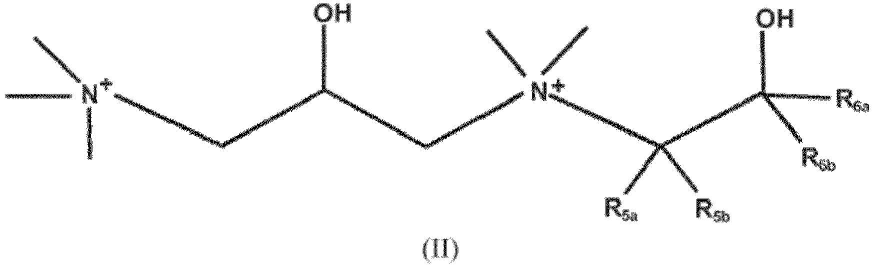 wobei:
R₅ₐ Ethyl ist, R_{5b} -CH₂OH ist und R₆ₐ und R_{6b} jeweils H sind.

4. Ein Verfahren zum Bereitstellen von Feuchtigkeitswahrung in einer Körperpflegezusammensetzung, das die Zugabe der Verbindungen der Ansprüche 1-3 in die Körperpflegezusammensetzung beinhaltet.

5. Eine Haarpflegezusammensetzung, die eine beliebige der Verbindungen der Ansprüche 1-3 enthält.

6. Eine Hautpflegezusammensetzung, die eine beliebige der Verbindungen der Ansprüche 1-3 enthält.

7. Eine Sonnenpflegezusammensetzung, die eine beliebige der Verbindungen der Ansprüche 1-3 enthält.

## Revendications

1. Un composé, incluant des sels, de la Formule (I) : 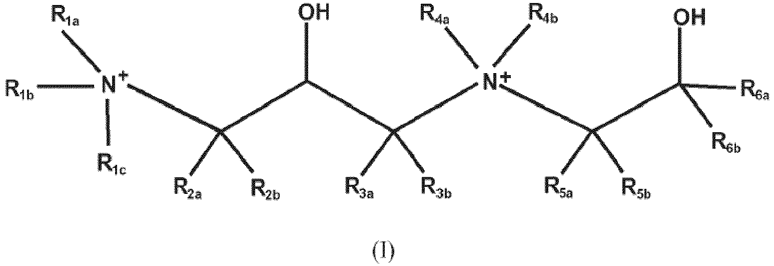 où :
R₁ₐ, R_{1b}, R_{1c}, R₂ₐ, R_{2b}, R₃ₐ, R_{3b}, R₅ₐ, R_{5b}, R₆ₐ, et R_{6b}, sont, indépendamment, H, un alkyle en C1 à C6 facultativement substitué, ou R₂ₐ et R_{2b}, R₃ₐ et R_{3b}, ou R₅ₐ et R_{5b}, peuvent coopérer pour former un cycloalkyle ; et
R₄ₐ et R_{4b} sont chacun -CH₂CH(OH)CH₂OH, ou R₄ₐ est -(CH₂)₃CH₃ et R_{4b} est -CH₂CH₂OH,
à condition qu'au moins l'un de R₄ₐ, R_{4b}, R₅ₐ, R_{5b}, R₆ₐ, et R_{6b} soit un alkyle en C1 à C6 substitué par au moins un groupe hydroxy ou un groupe amino où l'expression « facultativement substitué » signifie que les groupes en question sont soit non substitués, soit substitués par un ou plusieurs groupes, radicaux ou parties, sélectionnés parmi un halogène, un hydroxy, un amino ou un carboxy, et le terme « amino » inclut un amino substitué en sus par un alkyle en C1 à C3.

2. Le composé de la revendication 1, où R₁ₐ, R_{1b}, et R_{1c} sont chacun un méthyle.

3. Le composé, incluant des sels, ayant la Formule (II) : 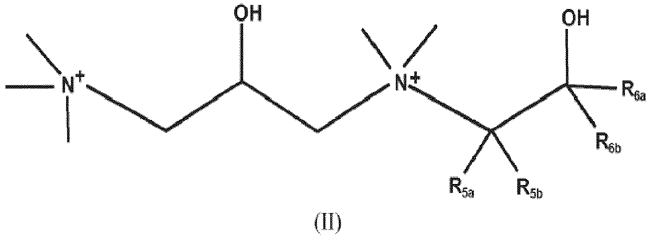 où :
R₅ₐ est un éthyle, R_{5b} est -CH₂OH, et R₆ₐ et R_{6b} sont chacun H.

4. Une méthode pour apporter une propriété humectante dans une composition de soins personnels, comprenant l'ajout des composés des revendications 1 à 3 dans la composition de soins personnels.

5. Une composition de soins capillaires contenant n'importe lesquels des composés des revendications 1 à 3.

6. Une composition pour le soin de la peau contenant n'importe lesquels des composés des revendications 1 à 3.

7. Une composition solaire contenant n'importe lesquels des composés des revendications 1 à 3.
